# NEUE EUROPÄISCHE PATENTSCHRIFT

(11) **EP 0 191 182 B2**
(45) Veröffentlichungstag und Bekanntmachung des Hinweises auf die Entscheidung über den Einspruch: **14.09.1994**
(45) Hinweis auf die Patenterteilung: 22.03.1989
(21) Anmeldenummer: 85115868.3
(22) Anmeldetag: 12.12.1985
(51) Int. Cl.: A61F 2/36

(54) **Knochenimplantat**
Bone implant
Implant osseux

(30) Priorität: 07.02.1985 CH 559/85
(43) Veröffentlichungstag der Anmeldung: 20.08.1986
(73) Patentinhaber: GEBRÜDER SULZER AKTIENGESELLSCHAFT, CH-8401 Winterthur (CH)
(72) Erfinder: Frey, Otto, CH-8400 Winterthur (CH); Semlitsch, Manfred, Dr., CH-8400 Winterthur (CH)

(56) Entgegenhaltungen:
- EP-A- 0 041 591
- EP-A- 0 065 481
- DE-A- 2 726 297
- FR-A- 2 429 589
- GB-A- 1 409 053
- US-A- 4 163 292

## Beschreibung

Die Erfindung betrifft ein Knochenimplantat aus Metall, insbesondere Gelenkendoprothese, teilweise aus einem Hohlkörper bestehend, wobei der Hohlkörper vorzugsweise eine unregelmässige geometrische Form und/oder eine profilierte Oberfläche aufweist.

Um vor allem bei Gelenkendoprothesen an Gewicht zu sparen, ist es bekannt, derartige Prothesen mindestens teilweise als geschlossene Hohlräume auszubilden (FR-A-2 021 313). Wie die massiven Prothesenteile werden die hohlen bisher aus Schmiede- und/oder Gussteilen hergestellt, wobei die geschlossenen Hohlkörper durch Zusammenschweissen einzelner Formteile gefertigt werden. Als Beispiel sei ein Gelenkkopf für eine Femurkopfprothese erwähnt, der aus einer Hohlkugel aus Gusswerkstoff und einer damit verschweissten Hülse aus einem Schmiedewerkstoff besteht vgl. die unter Art. 54(3) EPÜ fallende EP-A-0 172 262). Die herstellung und Bearbeitung der bisherigen Prothesen, die mindestens teilweise einen Hohlkörper bilden, ist daher - besonders wenn es sich z.B. um Verankerungsschäfte mit einer beispielsweise aus Rippen bestehenden Struktur handelt - teuer und aufwendig.

Weiterhin ist aus der EP-A-0 115 564 ein Knochenimplantat bekannt, das aus Blech hergestellt ist; dieses Implantat umgibt als Einsteckhülse den Verankerungsschaft von aus Kunststoff gefertigten Kleingelenken. Da die Form der Einsteckhülse relativ einfach ist, bereitet die Herstellung keine Schwierigkeiten und erfordert keinen grossen Aufwand.

Aufgabe der Erfindung ist es, die Herstellung von Knochenimplantaten, insbesondere von Gelenkendoprothesen, zu vereinfachen, ohne dabei eine unzulässige Einbusse an den geforderten mechanischen Eigenschaften in Kauf zu nehmen.

Diese Aufgabe wird mit der vorliegenden Erfindung dadurch gelöst, dass die Form und Oberfläche gestaltenden Begrenzungen des Hohlkörpers aus geschlossenem Blech, vorzugsweise aus einem superplastischen Werkstoff, gefertigt sind, das auf seinem ganzen Umfang mit einem festigkeitserhöhenden Grundkörper gasdicht verschweisst ist.

Das dünne Blech, dessen Dicke mit Vorteil mindestens 1 mm beträgt, lässt sich durch eine der für die Verformung von Blechen bekannten Verfahren leicht und einfach auch bei komplizierten Aussenformen in seine endgültige Gestalt bringen, ohne dass nachträgliche mechanische Bearbeitungen notwendig werden. Ein zusätzlicher Vorteil der Erfindung ergibt sich durch eine Gewichtsreduktion gegenüber gleichartigen massiven Konstruktionen.

Die die Form und Oberfläche gestaltenden Bleche sind dabei als geschlossene Hohlkörper ausgebildet.

Als Material für die formgestaltenden Bleche können Implantatwerkstoffe dienen, die sich zu und als Bleche ausreichender Festigkeit verformen lassen; bevorzugte Materialien sind dabei Alpha/Beta-Titanlegierungen, da diese Titanlegierungen superplastisch verformbar sind.

Bilden die Bleche in Verbindung mit einem Grundkörper einen geschlossenen Hohlkörper, so kann dieser mit leichten Materialien hoher Steifigkeit, beispielsweise Kunststoffschäumen, wie Polyäthylen u.a., oder mit einem elastisch oder plastisch verformbaren volumenstabilen Material, wie Silikonkautschuk oder Epoxy-Harz, gefüllt sein. Diese Füllungen erhöhen dabei die Festigkeit und Formstabilität der Bleche.

Die Stabilität der endgültig geformten Bleche läßt sich erhöhen, wenn die Form durch mindestens zwei Bleche gestaltet ist, die schichtartig übereinander angeordnet sind. Dabei kann das äußere Blech, das sich möglichst an Form und Oberfläche der ihm gegenüberliegenden Knochenwand anpassen sollte, mit Vorteil dünner und leichter verformbar sein als das «dahinter liegende» innere, das im wesentlichen nur eine Stütze für das äußere Blech bildet.

Im folgenden wird die Erfindung anhand von Ausführungsbeispielen im Zusammenhang mit der Zeichnung näher erläutert.
Fig. 1 ist eine Ansicht der lateralen Schmalseite des geraden Schaftes einer Hüftgelenkprothese, wobei der proximale Bereich des Schaftes im Schnitt dargestellt ist.;
Fig. 2a und 2b geben den ungeformten und den geformten Querschnitt des Schaftes nach Fig. 1 auf der Höhe des Schnitts 11-11 wieder;
Fig. 3 schließlich stellt einen in der Längsmittelebene «halbierten» Querschnitt eines Schaftes mit zwei schichtartig aufeinander gelegten formgestaltenden Blechen dar.

Der sich von seinem distalen Ende 1 aus konisch erweiternde Schaft 2 einer nicht weiter dargestellten Femurkopfprothese ist über etwa seiner Höhe - vom Ende 1 aus gesehen - wie bisher üblich als massives Blatt ausgebildet.

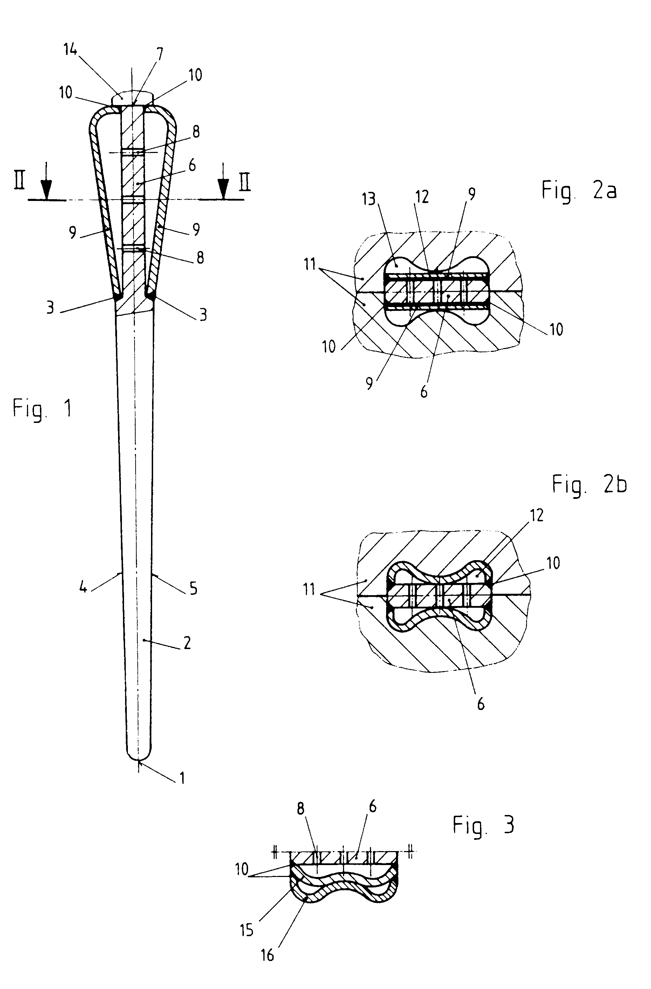

Mit einem Absatz 3 gehen seine sich konisch erweiternden Seiten 4 und 5 in einen plattenförmigen Grundkörper 6 über, der sich bis zum proximalen Schaftende 7 erstreckt, über dem in Fig. 1 der räumlich gegen das Schaftende 7 versetzte Prothesenhalsansatz 14 sichtbar ist. Auf den Grundkörper 6, der mit Durchbrüchen 8 versehen ist, sind Form und Oberfläche des proximalen Schaftteils gestaltende Bleche 9 aufgeschweißt, deren schematisch dargestellte Querschnittsform Fig. 2b zeigt.

Die Bleche 9 bestehen im vorliegenden Beispiel aus einem superplastischen Material, nämlich aus einer Alpha/Beta-Titanlegierung vom Typ Ti-6AI-4V, das durch Verformung in seine endgültige Gestalt übergeführt worden ist.

Eine Herstellungsart für ihre Verformung sei nunmehr in Verbindung mit Fig. 2a und 2b beschrieben. Die Bleche 9 werden zunächst auf dem ganzen Umfang mit Hilfe von Schweißnähten 10 gasdicht mit dem Grundkörper 6 verbunden. In einem nächsten Schritt wird mindestens der von den Blechen 9 bedeckte proximale Bereich des Schaftes 2 in eine zweiteilige Hohlform 11 eingelegt, deren Hohlraum 13 der negativen Gestalt der endgültigen Form des Schaftes 2 entspricht. Nun wird ein fluides Druckmedium über nicht gezeigte Leitungen und Anschlüsse in den in Fig. 2a gezeigten Hohlraum 12 zwischen Grundkörper 6 und Blechen 9 eingepreßt. Dieses bewirkt eine Verformung der in superplastischem Zustand befindlichen Bleche 9, die dabei die Gestalt der Begrenzungen des Formhohlraumes 13 annehmen.

Bei der Ausführungsform der Erfindung nach Fig. 3 sind an einen Grundkörper 6 für die Formung einer Seite der blattartigen Schaftoberfläche zwei schichtartig übereinander angeordnete Bleche 15, 16 vorgesehen. Von diesen ist das innere 15 mit dem Grundkörper 6 verschweißt, während das äußere 16 im vorliegenden Beispiel auf das innere Blech 15 aufgeschweißt ist. Selbstverständlich kann bei anderen Konstruktionen das äußere Blech 16 auch das innere 15 unvollständig umfassen und ebenfalls am Grundkörper 6 befestigt sein. Wie erwähnt, dient das äußere Blech 16, das leichter verformbar - und daher häufig dünner - als das innere 15 ist, zur Anpassung der Prothesenform an den Knochenhohlraum. Das innere Blech 15 hat die Aufgabe, die Stabilität des äußeren 16 zu erhöhen.

Die Formung auch der Bleche 15, 16 kann beispielsweise auf die bereits beschriebene Art durchgeführt werden, wobei - falls erforderlich - in dem inneren Blech 15 nicht gezeigte Durchtrittsöffnungen für das Druckmedium vorhanden sein können.

## Patentansprüche

1. Knochenimplantat aus Metall, insbesondere Gelenkendoprothese, teilweise aus einem Hohlkörper bestehend, wobei der Hohlkörper vorzugsweise eine unregelmässige geometrische Form und/oder eine profilierte Oberfläche aufweist, dadurch gekennzeichnet, dass die Form und Oberfläche gestaltenden Begrenzungen (9, 15, 16) des Hohlkörpers aus geschlossenem Blech sind, das auf seinem ganzen Umfang mit einem festigkeitserhöhenden Grundkörper (6) gasdicht verschweisst ist.

2. Knochenimplantat nach Anspruch 1, dadurch gekennzeichnet, daß die Bleche (9, 15, 16) aus einem superplastischen Werkstoff bestehen.

3. Knochenimplantat nach Anspruch 1 oder 2, dadurch gekennzeichnet, daß die Bleche (9, 15, 16) mindestens Teil eines geschlossenen Hohlkörpers sind, der mit einem elastischen volumenstabilen Material gefüllt ist.

4. Knochenimplantat nach einem der Ansprüche 1 bis 3, dadurch gekennzeichnet, daß die Form durch mindestens zwei Bleche (15, 16) gestaltet ist, die schichtartig übereinander angeordnet sind.

5. Knochenimplantat nach Anspruch 4, dadurch gekennzeichnet, daß die geschichteten Bleche (15, 16) unterschiedliche Verformungswiderstände haben.

6. Knochenimplantat nach einem der vorhergehenden Ansprüche, dadurch gekennzeichnet, daß die Wanddicke der Bleche (9, 15, 16) mindestens 1 mm beträgt.

## Claims

1. A bone implant of metal, more particularly a joint endoprosthesis, partly in the form of a hollow member, the same preferably having an irregular geometric shape and/or a profiled surface, characterised in that the boundaries (9, 15, 16) of the hollow member which define its shape and surface are made of closed sheet metal welded in gas-tight manner over its entire periphery to a strength-enhancing parent member (6).

2. An implant according to claim 1, characterised in that the sheet metal members (9, 15, 16) are made of a superplastic substance.

3. An implant according to claim 1 or 2, characterised in that the sheet metal members (9, 15, 16) are at least part of a closed hollow member which is filled with a resilient stable-volume material.

4. An implant according to any of claims 1 - 3, characterised in that the shape is defined by at least two sheet metal members (15, 16) disposed one above another in layered form.

5. An implant according to claim 4, characterised in that the layered sheet metal members (15, 16) have different resistances to deformation.

6. An implant according to any of the previous claims, characterised in that the wall thickness of the sheet metal members (9, 15, 16) is at least 1 mm.

## Revendications

1. Implant osseux en métal, notamment endopro- thèse articulée, constitué en partie d'un corps creux, ce corps creux présentant, de préférence, une forme géométrique irrégulière et/ou une surface profilée, caractérisé en ce que les délimitations (9, 15, 16) du corps creux, déterminant la forme et la surface, sont réalisées en tôle fermée qui, sur toute sa périphérie, est soudée, de manière étanche aux gaz, à un corps de base (6) accroissant la résistance mécanique.

2. Implant osseux selon la revendication 1, caractérisé en ce que les tôles (9, 15, 16) consistent en un matériau superplastique.

3. Implant osseux selon la revendication 1 ou 2, caractérisé en ce que les tôles (9, 15, 16) constituent au moins une partie d'un corps creux fermé qui est comblé avec un matériau élastique à volume stable.

4. Implant osseux selon l'une des revendications 1 à 3, caractérisé en ce que la forme est déterminée par au moins deux tôles (15, 16) qui sont superposées sous forme de couches.

5. Implant osseux selon la revendication 4, caractérisé en ce que les tôles (15, 16) disposées en couches possèdent des résistances à la déformation différentes.

6. Implant osseux selon l'une des revendications précédentes, caractérisé en ce que l'épaisseur de paroi des tôles (9, 15, 16) mesure au moins 1 mm.
